(19) **Europäisches Patentamt / European Patent Office / Office européen des brevets**

(11) **EP 4 543 299 B1**

(12) # EUROPEAN PATENT SPECIFICATION

(45) Date of publication and mention
of the grant of the patent:
**05.08.2026   Bulletin 2026/32**

(21) Application number: **23732445.4**

(22) Date of filing: **08.06.2023**

(51) International Patent Classification (IPC):
***A61B 8/00*** (2006.01)

(52) Cooperative Patent Classification (CPC):
**A61B 8/4236; A61B 8/0858; A61B 8/486;
A61B 8/5223; A61B 8/5292; A61M 16/024;
G16H 20/40; G16H 30/20; G16H 30/40;
G16H 40/63; G16H 50/20; G16H 50/30;**
A61B 5/0816; A61M 2016/0027; A61M 2016/0036

(86) International application number:
**PCT/EP2023/065328**

(87) International publication number:
**WO 2023/247195 (28.12.2023 Gazette 2023/52)**

(54) **DIAPHRAGM MEASUREMENT SYSTEM AND METHOD**

DIAPHRAGMA-MESSYSTEM UND -VERFAHREN

SYSTÈME ET PROCÉDÉ DE MESURE DU DIAPHRAGME

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:   **23.06.2022   US 202263354818 P**

(43) Date of publication of application:
**30.04.2025   Bulletin 2025/18**

(73) Proprietor: **Koninklijke Philips N.V.
5656 AE Eindhoven (NL)**

(72) Inventors:
 • **HAARTSEN, Jaap Roger
  5656 AG Eindhoven (NL)**
 • **BUIZZA, Roberto
  5656 AG Eindhoven (NL)**
 • **HENDRIKS, Cornelis Petrus
  5656 AG Eindhoven (NL)**
 • **WIEMKER, Rafael
  5656 AG Eindhoven (NL)**
 • **KOEHLER, Thomas
  5656AG Eindhoven (NL)**
 • **SABCZYNSKI, Jörg
  5656AG Eindhoven (NL)**
 • **POLKEY, Michael
  5656AG Eindhoven (NL)**

 • **WIEBERNEIT, Nataly
  5656AG Eindhoven (NL)**

(74) Representative: **Philips Intellectual Property &
Standards
High Tech Campus 34
5656 AE Eindhoven (NL)**

(56) References cited:
**US-A1- 2018 256 075**

 • **PATTARIN PIROMPANICH ET AL: "Use of
diaphragm thickening fraction combined with
rapid shallow breathing index for predicting
success of weaning from mechanical ventilator
in medical patients", JOURNAL OF INTENSIVE
CARE, BIOMED CENTRAL LTD, LONDON, UK,
vol. 6, no. 1, 2 February 2018 (2018-02-02), pages
1 - 7, XP021253205, DOI: 10.1186/
S40560-018-0277-9**

EP 4 543 299 B1

**(Cont. next page)**

- **MATAMIS DIMITRIOS ET AL: "Sonographic evaluation of the diaphragm in critically ill patients. Technique and clinical applications", INTENSIVE CARE MEDICINE, SPRINGER BERLIN HEIDELBERG, BERLIN/HEIDELBERG, vol. 39, no. 5, 24 January 2013 (2013-01-24), pages 801 - 810, XP037119915, ISSN: 0342-4642, [retrieved on 20130124], DOI: 10.1007/ S00134-013-2823-1**

- **DARARAT EKSOMBATCHAI ET AL: "The ratio of respiratory rate to diaphragm thickening fraction for predicting extubation success", BMC PULMONARY MEDICINE, BIOMED CENTRAL LTD, LONDON, UK, vol. 23, no. 1, 4 April 2023 (2023-04-04), pages 1 - 10, XP021316803, DOI: 10.1186/S12890-023-02392-W**

**Description**

The following relates generally to the respiratory therapy arts, mechanical ventilation arts, ventilator induced lung injury (VILI) arts, mechanical ventilation weaning arts, and related arts.

## BACKGROUND

A diaphragm thickening fraction (TFdi or TFDI) as measured by ultrasound (US) is widely recognized to evaluate diaphragm function to optimize ventilator support and weaning. The TFdi is defined as the percentage increase in diaphragm thickness relative to end-expiratory thickness during tidal breathing. The TFdi depends on diaphragmatic activity and reflects the diaphragm work of breathing (WoB) (i.e., the respiratory effort) (see, e.g., Vivier E, Mekontso Dessap A, Dimassi S, et al. "Diaphragm ultrasonography to estimate the work of breathing during non-invasive ventilation." Intensive Care Med 2012; 38: 796-803; Goligher EC, Fan E, Herridge MS, et al. "Evolution of diaphragm thickness during mechanical ventilation. Impact of inspiratory effort." Am J Respir Crit Care Med 2015; 192: 1080-1088).

Diaphragm thickness and strain can be assessed at the zone of apposition (ZA) during inspiration and expiration, using a linear high frequency transducer of 10-15 MHz. The zone of apposition is the chest wall area where the lower rib cage reaches the abdominal contents. The probe is positioned between the anteroaxillary and mid-axillary lines, perpendicular to the chest wall. With ultrasonic B-mode, the hemi-diaphragm is identified beneath the intercostal muscles as a hypoechogenic layer of muscle tissue located between two hyper-echogenic lines (the pleural line and the peritoneal line) (see, e.g., Fayssoil A, Behin A, Ogna A, et al. Diaphragm: Pathophysiology and Ultrasound Imaging in Neuromuscular Disorders. J Neuromuscul Dis. 2018). Diaphragmatic thickening is assessed by the thickening fraction (TFdi), calculated as the percentage inspiratory increase in the diaphragm thickness relative to end-expiratory thickness ($T_{ee}$) during tidal breathing, i.e., according to Equation 1:

$$\mathrm{TFdi} = \frac{T_{ei}-T_{ee}}{T_{ee}} * 100\% \qquad (1)$$

with $T_{ei}$ as the end-inspiratory thickness.

Some studies have evaluated the correlation between TFdi and respiratory effort (see, e.g., M. Umbrello et al. "Diaphragm ultrasound as indicator of respiratory effort in critically ill patients undergoing assisted mechanical ventilation: a pilot clinical study." Crit Care 19(1): 161, 2015). In this study, the authors found a correlation coefficient of R = 0.8 between TFdi and oesophageal pressure-time product and R = 0.7 between $TF_{di}$ and diaphragmatic pressure-time product. In another study (see, e.g., E. Oppersma et al. "Functional assessment of the diaphragm by speckle tracking ultrasound during inspiratory loading." J Appl Physiology, 123(5):1063-1070, 2017), at the zone of apposition the diaphragm strain can similarly be measured in real-time. For example, in this study, the functional assessment of the diaphragm by speckle tracking ultrasound during inspiratory loading was analyzed. The technique of speckle tracking ultrasound allows for the detection and tracking of diaphragmatic strain over time by analyzing acoustic markers called speckles. These speckles are formed by interference of ultrasound waves that are scattered from physical structures of a size comparable to the wavelength of the ultrasound waves. Both diaphragm strain and diaphragm strain rate were highly correlated to trans diaphragmatic pressure Pdi (strain $r^2 = 0.72$; strain rate $r^2 = 0.80$) and EAdi (strain $r^2 = 0.60$; strain rate $r^2 = 0.66$).

Moreover, the use of ultrasound to evaluate the respiratory muscle function (especially the diaphragm) is relatively new and remains infrequent due to the supposed difficulty in obtaining adequate measurements (see, e.g., Aarab Y, Jaber S, De Jong A, "Diaphragm Ultrasonography in ICU: Why, How, and When To Use It?" ICU Management & Practice, Volume 21 - Issue 3, 2021; Tuinman, P.R., Jonkman, A.H., Dres, M. et al. "Respiratory muscle ultrasonography: methodology, basic and advanced principles and clinical applications in ICU and ED patients-a narrative review." Intensive Care Med 46, 594-605 (2020)). Possible confounders reducing the reproducibility and accuracy of, for example, daily bed-side measurements are varying conditions such as a location of the US-probe at each measurement on the patient, an attitude (angulation) of the probe, a phase point in the patient's respiratory cycle, a manually exerted skin pressure of the probe, a user-chosen ultrasound settings, a thickening fraction varying over the extent of the diaphragm muscle, an effort to record, store, and process the data points manually, and so forth.

Diaphragm thickness and thickness fraction can be assessed using B-mode and M-mode imaging (see, e.g., Kalin BS, Gürsel G. "Does it make difference to measure diaphragm function with M mode (MM) or B mode (BM)?" J Clin Monit Comput. Vol. 34, 1247-1257, 2020). In case of M-mode first a 2D B-mode movie is recorded. From the images a single scan line is selected that intersects the diaphragm region of interest. Next, a time-motion image of that scan line is plotted from which the diaphragm thickness and thickness fraction can be determined.

Wearable ultrasound patches for non-invasive continuous assessment of diaphragm thickness can be used in several potential applications such as continuous atrophy detection, diaphragm dysfunction detection, accurate breathing rate detection, weaning prediction, asynchrony detection, and proportional ventilation (non-invasive NAVA).

Ideally, an ultrasound patch to measure the thickness (or thickening fraction) of the diaphragm is designed to be operated in M-mode, i.e. to make one or a few scan lines instead of a full two-dimensional (2D) image. This, to reduce cost, footprint, power consumption, and amount of data that needs to be processed and transferred. However, without a full 2D image it is difficult to identify the diaphragm in a single scan line.

The following section discloses certain improvements to overcome these problems and others.

Reference is made to the following paper: PATTARIN PIROMPANICH ET AL: "Use of diaphragm thickening fraction combined with rapid shallow breathing index for predicting success of weaning from mechanical ventilator in medical patients", JOURNAL OF INTENSIVE CARE, BIOMED CENTRAL LTD, LONDON, UK, vol. 6, no. 1, 2 February 2018 (2018-02-02), pages 1-7. This discloses a method for predicting successful ventilator weaning using diaphragm thickness and rapid shallow breathing index (RSBI).

Reference is further made to US 2018/256075 A1. This discloses a method for non-invasively monitoring the respiration of a patient based on ultrasound acquisition.

Reference is further made to the following paper: MATAMIS DIMITRIOS ET AL: "Sonographic evaluation of the diaphragm in critically ill patients. Technique and clinical applications", INTENSIVE CARE MEDICINE, SPRINGER BERLIN HEIDELBERG, BERLIN/HEIDELBERG, vol. 39, no. 5, 24 January 2013 (2013-01-24), pages 801-810. This discloses a method for sonographic evaluation of the diaphragm in critically ill patients.

## SUMMARY

In one aspect, a diaphragm measurement system includes at least one electronic processor programmed to perform a diaphragm measurement method including receiving ultrasound imaging data of a diaphragm of a patient over a time period encompassing multiple breaths; receiving respiration data of the patient over the time period; calculating a diaphragm thickness metric based on the received ultrasound imaging data of the diaphragm and the received respiration data; and displaying, on a display device, a representation of the calculated diaphragm thickness metric.

In another aspect, a diaphragm measurement method includes, with at least one electronic processor, receiving ultrasound imaging data of a diaphragm of a patient over a time period encompassing multiple breaths; receiving respiration data of the patient over the time period; calculating a diaphragm thickness metric based on the received ultrasound imaging data of the diaphragm and the received respiration data; and displaying, on a display device, a representation of the calculated diaphragm thickness metric.

One advantage resides in using a wearable ultrasound (US) patch to acquire US imaging data of a patient.

Another advantage resides in determining a location of a diaphragm of a patient from US imaging data.

Another advantage resides in determining a location of a diaphragm of a patient without a full US image.

Another advantage resides in controlling settings of a US patch to acquire US imaging data of a patient.

Another advantage resides in controlling settings of a mechanical ventilator based on US imaging data of a patient.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically shows an illustrative mechanical ventilation system in accordance with the present disclosure.
FIGURE 2 shows an example flow chart of operations suitably performed by the system of FIGURE 1.

## DETAILED DESCRIPTION

As used herein, the singular form of "a", "an", and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

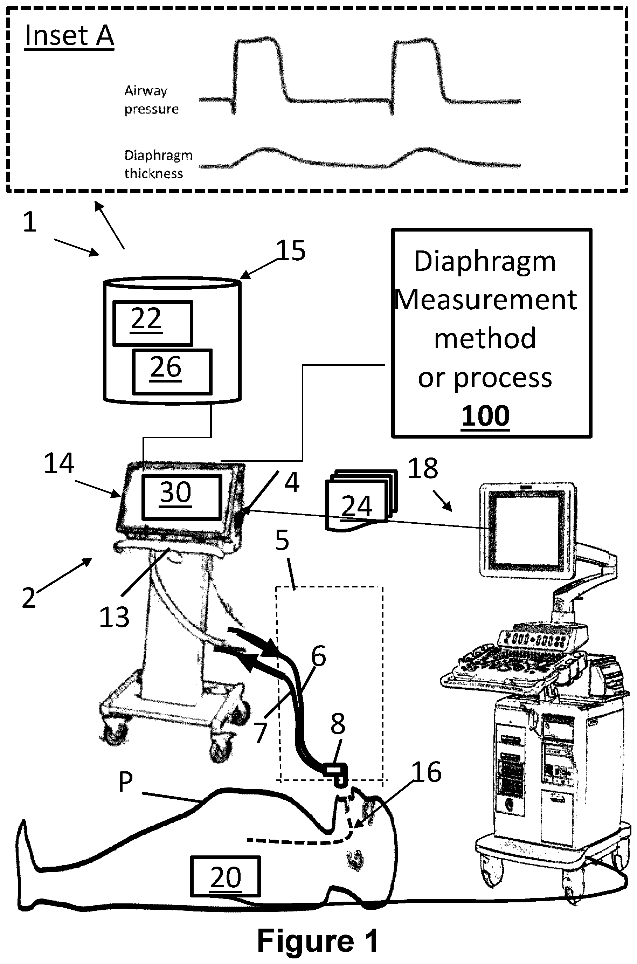

With reference to FIGURE 1, a diaphragm measurement device or system **1** is shown. A mechanical ventilator **2** is configured to provide ventilation therapy to an associated patient **P** is shown. As shown in FIGURE 1, the mechanical ventilator **2** includes an outlet **4** connectable with a patient breathing circuit **5** to delivery mechanical ventilation to the patient **P.** The patient breathing circuit **5** includes typical components for a mechanical ventilator, such as an inlet line **6,** an optional outlet line **7** (this may be omitted if the ventilator employs a single-limb patient circuit), a connector or port **8** for connecting with an endotracheal tube (ETT) **16,** and one or more breathing sensors (not shown), such as a gas flow meter, a pressure sensor, end-tidal carbon dioxide ($etCO_2$) sensor, and/or so forth. The mechanical ventilator **2** is designed to deliver air, an air-oxygen mixture, or other breathable gas (supply not shown) to the outlet **4** at a programmed pressure and/or flow rate to ventilate the patient via an ETT. The mechanical ventilator **2** also includes at least one electronic processor or controller **13** (e.g., an electronic processor or a microprocessor), a display device **14,** and a non-transitory computer readable medium **15** storing instructions executable by the electronic controller **13.**

FIGURE 1 diagrammatically illustrates the patient **P** intubated with an ETT **16** (the lower portion of which is inside the patient **P** and hence is shown in phantom). The connector or port **8** connects with the ETT **16** to operatively connect the mechanical ventilator **2** to deliver breathable air to the patient **P** via the ETT **16.** The mechanical ventilation provided by the mechanical ventilator **2** via the ETT **16** may be therapeutic for a wide range of conditions, such as various types of pulmonary conditions like emphysema or pneumonia, viral or bacterial infections impacting respiration such as a COVID-19 infection or severe influenza, cardiovascular conditions in which the patient **P** receives breathable gas enriched with oxygen, or so forth.

FIGURE 1 shows the patient **P** already intubated. That is, FIGURE 1 shows the patient after a tracheal intubation has been performed to insert the ETT **16** into the patient. However, to safely perform the tracheal intubation, the anesthesiologist or other qualified medical professional first performs an assessment of the patient **P** to select the ETT size of the ETT **16,** and then inserts an ETT of the selected size into the patient **P** by a tracheal intubation procedure.

FIGURE 1 also shows a medical imaging device **18** (also referred to as an image acquisition device, imaging device, and so forth). As primarily described herein, the medical imaging device **18** comprises an ultrasound (US) medical imaging device **18.** In other embodiments, the image acquisition device **18** can be a Computed Tomography (CT) image acquisition device, a C-arm imager, or other X-ray imaging device; Magnetic Resonance (MR) image acquisition device; or a medical imaging device of another modality. As described herein, the medical imaging device **18** is used to acquire images of the patient **P.** In some embodiments, the medical imaging device **18** can comprise a wearable US imaging device **18.** In other embodiments, the medical imaging device **18** can comprise a US imaging probe **18.**

In a more particular example, the medical imaging device **18** includes an ultrasound patch **20** that is wearable by the patient **P** (e.g., on the abdomen or chest of the patient **P** in position to image the diaphragm of the patient, as shown in FIGURE 1). The US patch **20** comprises one or more transducers, and is positioned to acquire US imaging data (i.e., US images) **24** of the diaphragm of the patient **P.** For example, the US patch **20** is configured to acquire imaging data of a diaphragm of the patient **P,** and more particularly US imaging data related to a thickness of the diaphragm of a patient **P** during inspiration and expiration while the patient **P** undergoes mechanical ventilation therapy with the mechanical ventilator **2.** The electronic processor **13** controls the ultrasound imaging device **18** to receive the ultrasound imaging data **24** of the diaphragm of the patient **P** from the handheld US patch **20.** In other embodiments, the electronic processor **13** can be implemented in the ultrasound imaging device **18** and can be configured to control operation of both the ultrasound imaging device **18** and/or the mechanical ventilator **2.** In further embodiments, the electronic processor **13** can be implemented in a separate electronic processing device (not shown), for example a computer, a smart tablet, and so forth.

In some embodiments, the non-transitory computer readable medium **15** stores an artificial neural network (ANN) model **22** configured to determine a diaphragm thickness metric based on the ultrasound imaging data **24** and respiration data of the patient. The non-transitory computer readable medium **15** also stores instructions executable by the electronic controller **13** to perform a diaphragm measurement method or process **100.**

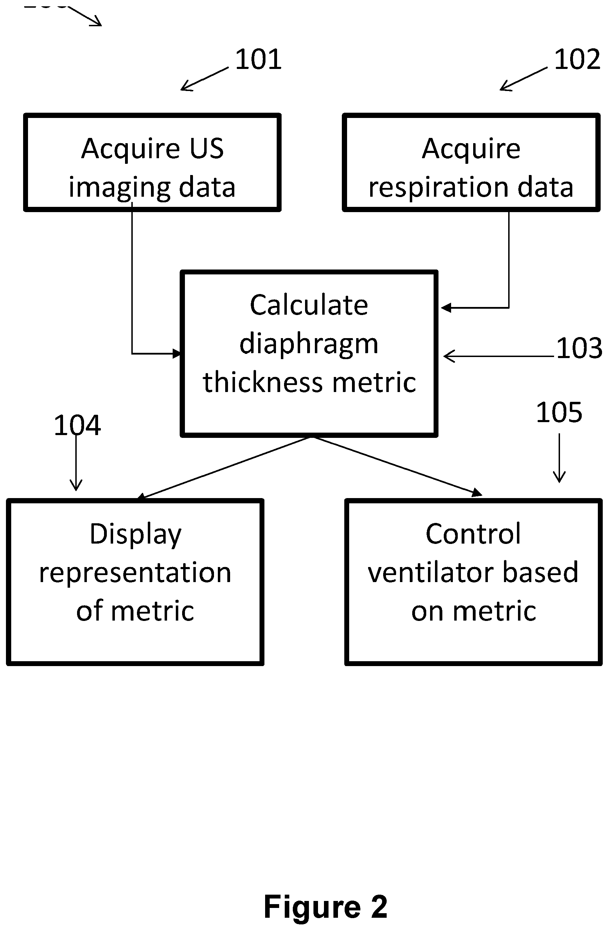

With reference to FIGURE 2, and with continuing reference to FIGURE 1, an illustrative embodiment of the diaphragm measurement method **100** is diagrammatically shown as a flowchart. At an operation **101,** the US imaging data **24** of the diaphragm of the patient **P** is received over a time period encompassing multiple breaths. For example, the electronic processor **13** controls the ultrasound imaging patch **20** to obtain the ultrasound imaging data **24** of the diaphragm of the patient **P.**

At an operation **102,** respiration data of the patient is received over the time period. The respiration data can be, for example, airway pressure (from a pressure sensor), airway flow (from an air flow sensor), or an output of a respiration monitor (such as a respiration belt worn by the patient **P**).

At an operation **103,** a diaphragm thickness metric can be calculated based on the received US imaging data **24** of the diaphragm of the patient **P** and the received respiration data. In one example, the diaphragm thickness metric includes a diaphragm thickening ratio indicative of a diaphragm thickness during inspiration relative to a diaphragm thickness during expiration. In another example, the diaphragm thickness metric includes a mean diaphragm thickness over multiple respiratory cycles.

At an operation **104,** a representation **30** of the calculated diaphragm thickness metric is displayed on the display device **14** of the mechanical ventilator **2**. In some embodiments, at an operation **105,** the mechanical ventilator **2** can be controlled to adjust one or more parameters of the mechanical ventilation therapy delivered to the patient based on the calculated diaphragm thickness metric.

In some embodiments, the US imaging data **24** comprises M-mode US imaging data. For example, the diaphragm can be automatically detected in a single channel ultrasound echo pattern, thereby making use of the correlation between airway pressure and diaphragm thickness of actively breathing patients receiving pressure support ventilation, or pressure or volume controlled ventilation. For example, for pressure support ventilation mode airway pressure correlates with diaphragm thickness, as shown in Inset A of FIGURE 1. In such embodiments, the calculating of the diaphragm thickness metric based on the received M-mode ultrasound imaging data **24** of the diaphragm and the received respiration data includes identifying a component of the M-mode ultrasound imaging data **24** corresponding to the diaphragm of the patient **P** based on the respiration data of the patient **P,** and calculating the diaphragm thickness metric based on the identified component of the M-mode ultrasound imaging data **24** corresponding to the diaphragm of the patient **P.** To do so, hyper-echogenic lines in the M-mode ultrasound imaging data **24** are identified, and grouped into pair of hyper-echogenic lines. For example, the hyper-echogenic lines can be identified by thresholding, edge-detection, or any other suitable technique. Alternatively, this is done on the raw ultrasound data **24** before it is converted into a grayscale image by identifying the high amplitude parts in the echo pattern (i.e. the strong echoes reflected from hyper-echogenic structures). The pairs of the hyper-echogenic lines represent possible boundaries of the diaphragm. Preferably, adjacent lines are grouped together since the diaphragm should appear as a hypo-echogenic layer of muscle tissue located between two adjacent hyper-echogenic lines, namely the pleural line and the peritoneal line.

For each pair of hyper-echogenic lines, a distance between the hyper-echogenic lines of the pair is determined as a function of time, and a correlation between the determined distance between the hyper-echogenic lines of the pair as a function of time and the respiration data of the patient **P** is determined. The component of the M-mode ultrasound imaging data **24** corresponding to a diaphragm of the patient **P** is identified as one of the pairs of hyper-echogenic lines based on the determined correlations. Since for pressure support ventilation airway pressure correlates with diaphragm thickening, the pair with the largest correlation coefficient is selected as being the two hyper-echoic lines in between which the diaphragm is located. For example, a respiration rate of the patient over the time period is identified from the respiration data of the patient **P,** and the M-mode ultrasound imaging data **24** is filtered using a bandpass filter **26** (for example, implemented in the non-transitory computer readable medium **15** of the mechanical ventilator **2**) with a passband centered at the identified respiration rate to extract the component of the M-mode ultrasound imaging data **24** corresponding to the diaphragm of the patient **P.**

In the case of multiple scan lines, the most optimal scan line could be selected by selecting the scan line with the largest correlation coefficient. Also, multiple scan lines could be selected (e.g., for those correlation coefficients exceeding a pre-determined threshold level) and a mean or median diaphragm thickness could be calculated. If the correlation coefficient(s) do not exceed a pre-determined threshold level the user/caregiver may be warned (e.g., to reposition the patch **20** or to check for partial or complete (paralyzed) diaphragm dysfunction). The procedure could be repeated on a regular (e.g., daily) basis.

In some embodiments, the US imaging data **24** of the diaphragm of the patient **P** is received over a time period encompassing multiple breaths, for example, during inspiration and expiration while the patient **P** undergoes mechanical ventilation therapy with the mechanical ventilator **2**. In such embodiments, the electronic controller **13** is configured to determine a phase shift between the bandpass-filtered M-mode ultrasound imaging data **24** and the respiration data of the patient **P.** A patient-ventilator asynchrony is determined based on the phase shift, and an indication of the determined patient-ventilator asynchrony is displayed on the display device **14** of the mechanical ventilator **2.** To do so, the US imaging data **24** and the respiration data are acquired, and a frequency of the mechanical ventilator **2** is determined. The US imaging time motion data is filtered, and the phase shift between the signal of the muscle thickening from the US time motion data and the ventilator pressure signal is determined.

In some embodiments, the diaphragm measurement method **100** can be repeated for successive sessions based on adjustments to the system **1.** In one example, one or more settings of the mechanical ventilator **2** are adjusted, and the method **100** is repeated for each adjustment. For example, the diaphragm is identified by changing ventilator settings and making use of the negative correlation between the diaphragm thickening fraction and a pressure support level actively breathing patients receiving pressure support to identify the diaphragm in the echo pattern. To do so, a ventilator support level is set. Hyper-echogenic lines in the time motion data are identified, and a distance between each pair of adjacent high amplitude parts is determined. For each breath, the time stamps of end-of-inspiration and end-of-expiration are determined from the pressure or flow waveforms. For each pair, the thickness fraction is calculated using the time stamps. These operations are repeated for several support levels. For each pair, the correlation coefficient between support level and maximum thickness is determined. The pair with largest correlation coefficient is selected as being the two hyper-echogenic lines between which the diaphragm is located.

In the case of multiple scan lines, the optimal scan line could be selected by repeating these operations for each scan line and selecting the scan line with the largest correlation coefficient. Also, multiple scan lines could be selected e.g. for those correlation coefficients exceeding a pre-determined threshold level, and a mean or median diaphragm thickness could be calculated. In addition, if the correlation coefficient(s) don't exceed a pre-determined threshold level the user/caregiver may be warned e.g. to reposition the patch **20**.

In another example, one or more settings of the ultrasound patch **20** (such as frequency, time-variable gain, steering angle, focus depth, and aperture size and position (i.e. selection of active elements in the ultrasound array) are adjusted, and the method **100** is repeated for each adjustment. The ultrasound patch **20** is equipped with a transducer array that allows for steering, several scan lines could be recorded by sweeping the angle. The optimal scan line could be selected, or an average thickness (fraction) could be calculated. Similarly, optimal settings could be found for frequency, focus depth, etc.

In another embodiment, the calculating of the diaphragm thickness metric can include inputting the M-mode ultrasound imaging data **24** and the respiration data to the ANN model **22** to determine the diaphragm thickness metric based on the M-mode ultrasound imaging data **24** and the respiration data of the patient **P.** The correlation between pressure and diaphragm thickness is learned implicitly during training of the ANN model **22.** To do so, the M-mode ultrasound imaging data **24** and the respiration data are acquired and input to the ANN model **22,** and the ANN model **22** is trained to reproduce the ground truth diaphragm.

In some embodiments, the mechanical ventilator (MV) **2** provides several types of waveforms (e.g. pressure, flow, work-of-breathing, volume, etc). For each mutual pair of the US-line and the MV-signal, the correlation is determined, and the optimally correlating selection of the MV-waveform is determined. In cases of partial or complete diaphragm dysfunction, under pressure or volume-controlled ventilation, the diaphragm thickness fraction can be negative due to passive stretching (see, e.g., Santana PV, Cardenas LZ, Albuquerque ALP, Carvalho CRR, Caruso P. Diaphragmatic ultrasound: a review of its methodological aspects and clinical uses. J Bras Pneumol. 2020 Nov 20). Also, other factors can lead to a negative diaphragm thickness fraction, such as a too high-pressure support level. Hence, when detecting a negative diaphragm thickness fraction, a warning could be given to the caregiver or ventilation settings could automatically be adapted.

While described in terms of mechanical ventilation therapy, the system **1** can be used in any suitable setting, including heart surgery, heart failure monitoring, asynchrony detection, home ventilation, bladder and prostate enlargement monitoring, carotid artery narrowing (e.g., stroke, cardiac output, A-fib, and so forth), heart muscle function and cardiac output in an acute care setting (i.e., heart attack), and so forth.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims.

## Claims

1. A diaphragm measurement system (1), comprising:
   at least one electronic processor (13) programmed to perform a diaphragm measurement method including:

   receiving (101) ultrasound imaging data of a diaphragm of a patient over a time period encompassing multiple breaths;
   receiving (102) respiration data of the patient over the time period;
   calculating (103) a diaphragm thickness metric based on the received ultrasound imaging data of the diaphragm and the received respiration data; and
   displaying (104), on a display device, a representation of the calculated diaphragm thickness metric.

2. The system of claim 1, wherein the diaphragm thickness metric includes a diaphragm thickening ratio indicative of a diaphragm thickness during inspiration relative to a diaphragm thickness during expiration.

3. The system of claim 1, wherein the diaphragm thickness metric includes a mean diaphragm thickness over multiple respiratory cycles.

4. The system of claim 1, further comprising:
   an ultrasound imaging patch wearable by the patient, wherein the at least one electronic processor controls the ultrasound imaging patch to obtain the ultrasound imaging data of the diaphragm of the patient.

5. The system of claim 1, wherein the ultrasound imaging data comprises M-mode ultrasound imaging data, and the calculating of the diaphragm thickness metric based on the received M-mode ultrasound imaging data of the diaphragm and the received respiration data includes:

identifying a component of the M-mode ultrasound imaging data corresponding to the diaphragm of the patient based on the respiration data of the patient; and
calculating the diaphragm thickness metric based on the identified component of the M-mode ultrasound imaging data corresponding to the diaphragm of the patient.

6. The system of claim 5, wherein the at least one electronic processor is programmed to identify the component of the M-mode ultrasound imaging data corresponding to the diaphragm of the patient by:

identifying hyper-echogenic lines in the M-mode ultrasound imaging data;
grouping the hyper-echogenic lines into pairs of hyper-echogenic lines;
for each pair of hyper-echogenic lines, determining a distance between the hyper-echogenic lines of the pair as a function of time;
for each pair of hyper-echogenic lines, determining a correlation between the determined distance between the hyper-echogenic lines of the pair as a function of time and the respiration data of the patient; and
identifying the component of the M-mode ultrasound imaging data corresponding to a diaphragm of the patient as one of the pairs of hyper-echogenic lines based on the determined correlations.

7. The system of claim 5, wherein identifying the component of the M-mode ultrasound imaging data corresponding to the diaphragm of the patient based on the respiration data of the patient includes:

identifying a respiration rate of the patient over the time period from the respiration data of the patient; and
filtering the M-mode ultrasound imaging data using a bandpass filter with a passband centered at the identified respiration rate to extract the component of the M-mode ultrasound imaging data corresponding to the diaphragm of the patient.

8. The system of claim 7, wherein the patient is receiving mechanical ventilation over the time period, and the electronic processor is further programmed to:

determine a phase shift between the bandpass-filtered M-mode ultrasound imaging data and the respiration data of the patient;
determine a patient-ventilator asynchrony based on the phase shift; and
display, on the display device, an indication of the determined patient-ventilator asynchrony.

9. The system of claim 1, wherein the respiration data of the patient comprises airway pressure, airway flow, or an output of a respiration monitor.

10. The system of claim 1, wherein the patient is receiving mechanical ventilation over the time period, and the electronic processor is programmed to:
adjust one or more settings of a mechanical ventilator and repeat the diaphragm measurement method for each adjustment.

11. The system of claim 1, wherein the electronic processor is programmed to:
adjust one or more settings of an ultrasound patch configured to acquire the ultrasound imaging data and repeat the diaphragm measurement method for each adjustment.

12. The system of claim 1, wherein the calculating of the diaphragm thickness metric based on the received ultrasound imaging data of the diaphragm and the received respiration data includes
inputting the ultrasound imaging data and the respiration data to an artificial neural network (ANN) model configured to determine the diaphragm thickness metric based on the ultrasound imaging data and the respiration data of the patient.

13. The system of claim 1, wherein the at least one electronic processor is configured to:
control an associated mechanical ventilator to adjust one or more parameters of mechanical ventilation therapy delivered to the patient based on the calculated diaphragm thickness metric.

14. The system of claim 13, further comprising:
a mechanical ventilator configured to deliver mechanical ventilation therapy to the patient.

15. A diaphragm measurement method (100) comprising, with at least one electronic processor:

receiving (101) ultrasound imaging data of a diaphragm of a patient over a time period encompassing multiple breaths;
receiving (102) respiration data of the patient over the time period;
calculating (103) a diaphragm thickness metric based on the received ultrasound imaging data of the diaphragm and the received respiration data; and
displaying (104), on a display device, a representation of the calculated diaphragm thickness metric.

**Patentansprüche**

1. Diaphragma-Messsystem (1), umfassend: mindestens einen elektronischen Prozessor, der so programmiert ist, dass er ein Diaphragma-Messverfahren (13) durchführt, das Folgendes einschließt:

   Empfangen (101) von Ultraschall-Bildgebungsdaten eines Diaphragmas eines Patienten über einen Zeitraum, der mehrere Atemzüge umfasst;
   Empfangen (102) von Atmungsdaten des Patienten über den Zeitraum;
   Berechnen (103) eines Diaphragma-Dickenmaßes basierend auf den empfangenen Ultraschall-Bildgebungsdaten des Diaphragmas und den empfangenen Atmungsdaten; und
   Anzeigen einer Darstellung des berechneten Diaphragma-Dickenmaßes auf einer Anzeigevorrichtung (104).

2. System nach Anspruch 1, wobei das Diaphragma-Dickenmaß ein Diaphragma-Verdickungsverhältnis einschließt, das eine Diaphragma-Dicke während Einatmung im Verhältnis zu einer Diaphragma-Dicke während Ausatmung angibt.

3. Vorrichtung nach Anspruch 1, wobei das Diaphragma-Dickenmaß eine mittlere Diaphragma-Dicke über mehrere Atemzyklen einschließt.

4. System nach Anspruch 1, weiter umfassend: ein Ultraschall-Bildgebungspflaster, das von dem Patienten getragen werden kann, wobei der mindestens eine elektronische Prozessor das Ultraschall-Bildgebungspflaster steuert, um die Ultraschall-Bildgebungsdaten des Diaphragmas des Patienten zu erhalten.

5. System nach Anspruch 1, wobei die Ultraschall-Bildgebungsdaten M-Mode-Ultraschall-Bildgebungsdaten umfassen und das Berechnen des Diaphragma-Dickenmaßes basierend auf den empfangenen M-Mode-Ultraschall-Bildgebungsdaten des Diaphragmas und den empfangenen Atmungsdaten Folgendes einschließt:

   Identifizieren einer Komponente der M-Mode-Ultraschall-Bildgebungsdaten, die dem Diaphragma des Patienten entspricht, basierend auf den Atmungsdaten des Patienten; und
   Berechnen des Diaphragma-Dickenmaßes basierend auf der identifizierten Komponente der M-Mode-Ultraschall-Bildgebungsdaten, die dem Diaphragma des Patienten entspricht.

6. System nach Anspruch 5, wobei der mindestens eine elektronische Prozessor so programmiert ist, dass er die dem Diaphragma des Patienten entsprechende Komponente der M-Mode-Ultraschall-Bildgebungsdaten identifiziert durch:

   Identifizieren von hyperechogenen Linien in den M-Mode-Ultraschall-Bildgebungsdaten;
   Gruppieren der hyperechogenen Linien in Paaren von hyperechogenen Linien;
   für jedes Paar hyperechogener Linien Bestimmen eines Abstands zwischen den hyperechogenen Linien des Paares als Funktion von Zeit;
   für jedes Paar hyperechogener Linien Bestimmen einer Korrelation zwischen dem bestimmten Abstand zwischen den hyperechogenen Linien des Paares als Funktion von Zeit und den Atmungsdaten des Patienten; und
   Identifizieren der Komponente der M-Mode-Ultraschall-Bildgebungsdaten, die dem Diaphragma des Patienten entspricht, als eines der Paare hyperechogener Linien basierend auf den bestimmten Korrelationen.

7. System nach Anspruch 5, wobei Identifizieren der Komponente der M-Mode-Ultraschall-Bildgebungsdaten, die dem Diaphragma des Patienten entspricht, basierend auf den Atmungsdaten des Patienten Folgendes einschließt:

   Bestimmen einer Atmungsfrequenz des Patienten über den Zeitraum aus den Atmungsdaten des Patienten; und
   Filtern der M-Mode-Ultraschall-Bildgebungsdaten unter Verwendung eines Bandpassfilters mit einem auf die bestimmte Atemfrequenz zentrierten Durchlassband, um die Komponente der M-Mode-Ultraschall-Bildgebungsdaten zu extrahieren, die dem Diaphragma des Patienten entspricht.

8. System nach Anspruch 7, wobei der Patient während des Zeitraums mechanische Beatmung erhält und der elektronische Prozessor weiter programmiert ist zum:

   Bestimmen einer Phasenverschiebung zwischen den bandpassgefilterten M-Mode-Ultraschall-Bildgebungsdaten und den Atmungsdaten des Patienten;
   Bestimmen einer Asynchronie zwischen Patient und Beatmungsgerät basierend auf der Phasenverschiebung; und
   Anzeigen auf der Anzeigevorrichtung einer Angabe der bestimmten Asynchronie zwischen Patient und Beatmungsgerät.

9. System nach Anspruch 1, wobei die Atmungsdaten des Patienten Atemwegsdruck, Atemwegsfluss oder eine Ausgabe eines Atmungsmonitors umfassen.

**10.** System nach Anspruch 1, wobei der Patient über den Zeitraum hinweg mechanische Beatmung erhält und der elektronische Prozessor programmiert ist zum:
Anpassen einer oder mehrerer Einstellungen eines mechanischen Beatmungsgeräts und Wiederholen des Diaphragma-Messverfahrens für jede Anpassung.

**11.** System nach Anspruch 1, wobei elektronische Prozessor programmiert ist zum:
Anpassen einer oder mehrerer Einstellungen eines Ultraschall-Bildgebungspflaster, das so konfiguriert ist, dass es die Ultraschall-Bildgebungsdaten erfasst, und Wiederholen des Diaphragma-Messverfahrens für jede Anpassung.

**12.** System nach Anspruch 1, wobei das Berechnen des Diaphragma-Dickenmaßes basierend auf den empfangenen Ultraschall-Bildgebungsdaten des Diaphragmas und den empfangenen Atmungsdaten Folgendes einschließt:
Eingeben der Ultraschall-Bildgebungsdaten und der Atmungsdaten in ein künstliches neuronales Netzwerk-(KNN)-Modell, das so konfiguriert ist, dass es die Diaphragma-Dicke basierend auf den Ultraschall-Bildgebungsdaten und den Atmungsdaten des Patienten bestimmt.

**13.** System nach Anspruch 1, wobei der mindestens eine elektronische Prozessor konfiguriert ist zum:
Steuern eines zugeordneten mechanischen Beatmungsgeräts, um einen oder mehrere Parameter der an den Patienten abgegebenen mechanischen Beatmungstherapie basierend auf dem berechneten Diaphragma-Dickenmaß anzupassen.

**14.** System nach Anspruch 13, weiter umfassend:
ein mechanisches Beatmungsgerät, das so konfiguriert ist, dass es an den Patienten eine mechanische Beatmungstherapie abgibt.

**15.** Diaphragma-Messverfahren (100), umfassend, mit mindestens einem elektronischen Prozessor:

Empfangen (101) von Ultraschall-Bildgebungsdaten eines Diaphragmas eines Patienten über einen Zeitraum, der mehrere Atemzüge umfasst;
Empfangen (102) von Atmungsdaten des Patienten über den Zeitraum;
Berechnen (103) eines Diaphragma-Dickenmaßes basierend auf den empfangenen Ultraschall-Bildgebungsdaten des Diaphragmas und den empfangenen Atmungsdaten; und
Anzeigen einer Darstellung des berechneten Diaphragma-Dickenmaßes auf einer Anzeigevorrichtung (104).

**Revendications**

**1.** Dispositif de mesure de diaphragme (1), comprenant :
au moins un processeur électronique (13) programmé pour mettre en œuvre un procédé de mesure de diaphragme incluant :

la réception (101) de données d'imagerie ultrasonore d'un diaphragme d'un patient sur une période donnée englobant de multiples respirations ;
la réception (102) de données respiratoires du patient sur la période donnée ;
le calcul (103) d'une métrique d'épaisseur de diaphragme sur la base des données d'imagerie ultrasonore reçues du diaphragme et des données respiratoires reçues ; et
l'affichage (104), sur un dispositif d'affichage, d'une représentation de la métrique d'épaisseur de diaphragme calculée.

**2.** Dispositif selon la revendication 1, dans lequel la métrique d'épaisseur de diaphragme inclut un rapport d'épaississement de diaphragme indicatif d'une épaisseur de diaphragme pendant l'inspiration par rapport à une épaisseur de diaphragme pendant l'expiration.

**3.** Dispositif selon la revendication 1, dans lequel la métrique d'épaisseur de diaphragme inclut une épaisseur moyenne de diaphragme sur de multiples cycles respiratoires.

**4.** Système selon la revendication 1, comprenant en outre :
un patch d'imagerie ultrasonore portable par le patient, dans lequel l'au moins un processeur électronique commande le patch d'imagerie ultrasonore pour obtenir les données d'imagerie ultrasonore du diaphragme du patient.

**5.** Système selon la revendication 1, dans lequel les données d'imagerie ultrasonore comprennent des données d'imagerie ultrasonore en mode M et le calcul de la métrique d'épaisseur de diaphragme sur la base des données d'imagerie ultrasonore en mode M reçues du diaphragme et les données respiratoires reçues incluent :

l'identification d'une composante des données d'imagerie ultrasonore en mode M correspondant au diaphragme du patient sur la base des données respiratoires du patient ; et
le calcul de la métrique d'épaisseur de diaphragme sur la base de la composante identifiée des données d'imagerie ultrasonore en mode M correspondant au diaphragme du patient.

**6.** Système selon la revendication 5, dans lequel l'au moins un processeur électronique est programmé pour identifier la composante des données d'imagerie ultrasonore en mode M correspondant au diaphragme du patient par :

identification des lignes hyperéchogènes dans les données d'imagerie ultrasonore en mode M ; regroupement des lignes hyperéchogènes en paires de lignes hyperéchogènes ; pour chaque paire de lignes hyperéchogènes, la détermination d'une distance entre les lignes hyperéchogènes de la paire en fonction du temps ; pour chaque paire de lignes hyperéchogènes, la détermination d'une corrélation entre la distance déterminée entre les lignes hyperéchogènes de la paire en fonction du temps et les données respiratoires du patient ; et identification de la composante des données d'imagerie ultrasonore en mode M correspondant à un diaphragme du patient comme l'une des paires de lignes hyperéchogènes sur la base des corrélations déterminées.

**7.** Système selon la revendication 5, dans lequel l'identification de la composante des données d'imagerie ultrasonore en mode M correspondant au diaphragme du patient sur la base des données respiratoires du patient inclut :

l'identification d'une fréquence respiratoire du patient sur la période donnée à partir des données respiratoires du patient ; et le filtrage des données d'imagerie ultrasonore en mode M à l'aide d'un filtre passe-bande avec une bande passante centrée sur la fréquence respiratoire identifiée pour extraire la composante des données d'imagerie ultrasonore en mode M correspondant au diaphragme du patient.

**8.** Système selon la revendication 7, dans lequel le patient reçoit une ventilation mécanique pendant la période donnée et le processeur électronique est en outre programmé pour :

déterminer un déphasage entre les données d'imagerie ultrasonore en mode M filtrées par passe-bande et les données respiratoires du patient ; déterminer une asynchronie patient-ventilateur sur la base du déphasage ; et afficher, sur le dispositif d'affichage, une indication de l'asynchronie patient-ventilateur déterminée.

**9.** Système selon la revendication 1, dans lequel les données respiratoires du patient comprennent la pression des voies respiratoires, le débit des voies respiratoires ou une sortie d'un moniteur respiratoire.

**10.** Système selon la revendication 1, dans lequel le patient reçoit une ventilation mécanique pendant la période donnée et le processeur électronique est programmé pour :
ajuster un ou plusieurs paramètres d'un ventilateur mécanique et répéter le procédé de mesure de diaphragme pour chaque ajustement.

**11.** Dispositif selon la revendication 1, dans lequel le processeur électronique est programmé pour :
ajuster un ou plusieurs réglages d'un patch ultrasonore configuré pour acquérir les données d'imagerie ultrasonore et répéter le procédé de mesure de diaphragme pour chaque ajustement.

**12.** Système selon la revendication 1, dans lequel le calcul de la métrique d'épaisseur de diaphragme sur la base des données d'imagerie ultrasonore du diaphragme reçues et des données respiratoires reçues inclut
l'entrée des données d'imagerie ultrasonore et des données respiratoires dans un modèle de réseau neuronal artificiel (RNA) configuré pour déterminer la métrique d'épaisseur de diaphragme sur la base des données d'imagerie ultrasonore et des données respiratoires du patient.

**13.** Système selon la revendication 1, dans lequel l'au moins un processeur électronique est configuré pour :
commander un ventilateur mécanique associé pour ajuster un ou plusieurs paramètres de la thérapie de ventilation mécanique administrée au patient sur la base de la métrique d'épaisseur de diaphragme calculée.

**14.** Système selon la revendication 13, comprenant en outre :
un ventilateur mécanique configuré pour administrer une thérapie de ventilation mécanique au patient.

**15.** Procédé de mesure de diaphragme (100) comprenant, avec au moins un processeur électronique :

la réception (101) de données d'imagerie ultrasonore d'un diaphragme d'un patient sur une période donnée englobant de multiples respirations ; la réception (102) de données respiratoires du patient sur la période donnée ; le calcul (103) d'une métrique d'épaisseur de diaphragme sur la base des données d'imagerie ultrasonore du diaphragme et des données res-

**EP 4 543 299 B1**

piratoires reçues ; et
l'affichage (104), sur un dispositif d'affichage, d'une représentation de la métrique d'épaisseur de diaphragme calculée.

**Inset A**

Airway pressure

Diaphragm thickness

1

15

Diaphragm Measurement method or process **100**

22

26

14

30

4

24

18

2

13

5

6

7

8

P

20

16

**Figure 1**

Figure 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018256075 A1

**Non-patent literature cited in the description**

- **VIVIER E** ; **MEKONTSO DESSAP A** ; **DIMASSI S et al.** Diaphragm ultrasonography to estimate the work of breathing during non-invasive ventilation. *Intensive Care Med*, 2012, vol. 38, 796-803
- **GOLIGHER EC** ; **FAN E** ; **HERRIDGE MS et al.** Evolution of diaphragm thickness during mechanical ventilation. Impact of inspiratory effort.. *Am J Respir Crit Care Med*, 2015, vol. 192, 1080-1088
- **FAYSSOIL A** ; **BEHIN A** ; **OGNA A et al.** Diaphragm: Pathophysiology and Ultrasound Imaging in Neuromuscular Disorders. *J Neuromuscul Dis.*, 2018
- **M. UMBRELLO et al.** Diaphragm ultrasound as indicator of respiratory effort in critically ill patients undergoing assisted mechanical ventilation: a pilot clinical study. *Crit Care*, 2015, vol. 19 (1), 161
- **E. OPPERSMA et al.** Functional assessment of the diaphragm by speckle tracking ultrasound during inspiratory loading.. *J Appl Physiology*, 2017, vol. 123 (5), 1063-1070
- **AARAB Y** ; **JABER S** ; **DE JONG A**. Diaphragm Ultrasonography in ICU: Why, How, and When To Use It?. *ICU Management & Practice*, 2021, vol. 21 (3)
- **TUINMAN, P.R** ; **JONKMAN, A.H.** ; **DRES, M. et al.** Respiratory muscle ultrasonography: methodology, basic and advanced principles and clinical applications in ICU and ED patients-a narrative review. *Intensive Care Med*, 2020, vol. 46, 594-605
- **KALIN BS** ; **GÜRSEL G**. Does it make difference to measure diaphragm function with M mode (MM) or B mode (BM)?. *J Clin Monit Comput*, 2020, vol. 34, 1247-1257
- Use of diaphragm thickening fraction combined with rapid shallow breathing index for predicting success of weaning from mechanical ventilator in medical patients. **PATTARIN PIROMPANICH et al.** JOURNAL OF INTENSIVE CARE. BIOMED CENTRAL LTD, 02 February 2018, vol. 6, 1-7
- Sonographic evaluation of the diaphragm in critically ill patients. Technique and clinical applications. **MATAMIS DIMITRIOS et al.** INTENSIVE CARE MEDICINE. SPRINGER BERLIN HEIDELBERG, 24 January 2013, vol. 39, 801-810
- **SANTANA PV** ; **CARDENAS LZ** ; **ALBUQUERQUE ALP** ; **CARVALHO CRR** ; **CARUSO P**. Diaphragmatic ultrasound: a review of its methodological aspects and clinical uses. *J Bras Pneumol.*, 20 November 2020